Europäisches Patentamt

European Patent Office

Office européen des brevets

(11) Veröffentlichungsnummer: **0 248 323**
**B1**

## EUROPÄISCHE PATENTSCHRIFT

(45) Veröffentlichungstag der Patentschrift:
07.02.90

(21) Anmeldenummer: 87107612.1

(22) Anmeldetag: 25.05.87

(51) Int. Cl.⁴: **A61B 6/02**, A61B 6/12,
H05G 1/70

(54) Röntgenortungseinrichtung.

(30) Priorität: 05.06.86 DE 3618869

(43) Veröffentlichungstag der Anmeldung:
09.12.87 Patentblatt 87/50

(45) Bekanntmachung des Hinweises auf die Patenterteilung:
07.02.90 Patentblatt 90/6

(84) Benannte Vertragsstaaten:
DE FR GB NL

(56) Entgegenhaltungen:
EP-A- 0 168 559
DE-A- 3 122 056
DE-A- 3 220 751
DE-B- 3 006 749

(73) Patentinhaber: Siemens Aktiengesellschaft,
Wittelsbacherplatz 2, D-8000 München 2(DE)

(72) Erfinder: Oppelt, Sylvester, Dipl.-Ing.(FH),
Greiffenbergstrasse 51, D-8600 Bamberg(DE)

## Beschreibung

Die Erfindung betrifft eine Röntgenortungseinrichtung mit zwei Röntgenstrahlenquellen zur Erzeugung zweier sich in einem Zielgebiet schneidender Röntgenstrahlenbündel und zwei Bildwandlern zur Erzeugung zweier Bildsignale und zwei daran angeschlossenen Monitoren. Derartige Einrichtungen dienen zur genauen Ortung von Körperteilen. Solch eine Einrichtung ist aus DE-A 3 220 751 bekannt.

In der DE-A 31 22 056 ist eine Vorrichtung beschrieben, die beispielsweise zur Ortung von Harnsteinen, Nierensteinen, Gallensteinen oder dergleichen bei einer Einrichtung zum Zertrümmern von im Körper eines Lebewesens befindlichen Konkrementen mit einem Stoßwellengenerator dient. In einer Fokussierungskammer wird als Stoßwellengenerator eine Stoßwelle z.B. durch Funkenentladung erzeugt, die auf das Konkrement konzentriert wird und es zerkleinert. Zur genauen Ortung des Konkrementes und Ausrichten desselben in den Brennpunkt der Fokussierungskammer ist die Einrichtung mit einer Röntgenuntersuchungsvorrichtung verbunden. Im Durchleuchtungsbetrieb werden die einzelnen Bilder eines Stereobildpaares in Bildspeicher einzeln oder über mehrere Bilder integriert im Fernsehtakt eingespeichert. Hierbei tritt aber das Problem auf, daß die Konkremente, insbesondere wenn sie klein sind, nur einen geringen Kontrastumfang aufweisen, so daß sie nur schwer erkennbar sind.

Die Erfindung geht von der Aufgabe aus, eine Röntgendiagnostikeinrichtung der eingangs genannten Art zu schaffen, bei der eine gute Erkennbarkeit von kleinen Teilen mit geringem Kontrastumfang gegeben ist.

Die Aufgabe wird erfindungsgemäß dadurch gelöst, daß das Bildsignal des ersten Bildwandlers einem Bildrechner zugeführt wird, der ein Bildsignal berechnet, das der Projektion des einen Röntgenstrahlenbündels in der Ebene des anderen Röntgenstrahlenbündels entspricht, und daß eine Additionsstufe zwischen dem zweiten Bildwandler und dem zweiten Monitor geschaltet ist, die das Ausgangssignal des Bildrechners und das des zweiten Bildwandlers mischt. Dadurch wird die Sichtbarkeit der kleinen Konkremente verstärkt.

Eine Berechnung bei der Wiedergabe auf zwei Monitoren kann erfolgen, wenn an den beiden Bildwandlern je eine Additionsstufe und je ein Bildrechner angeschlossen sind, deren Ausgänge mit der jeweils anderen Additionsstufe verbunden sind.

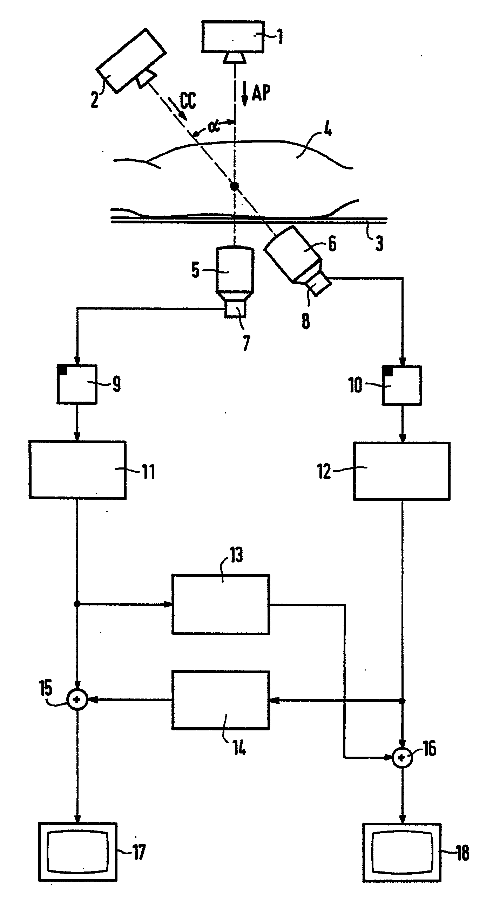

In der Figur ist eine Röntgeneinrichtung dargestellt, die zwei Röntgenröhren 1 und 2 aufweist, die Röntgenstrahlenbündel erzeugen, die einen auf einer Patientenliege 3 befindlichen Patienten 4 durchdringen und auf die Eingangsleuchtschirme von Röntgenbildverstärker 5 und 6 fallen. Die Röntgenröhre 1 und der Röntgenbildverstärker 5 können dabei derart angeordnet sein, daß der Zentralstrahl des Röntgenstrahlenbündels der Röntgenröhre 1 senkrecht auf den Patienten 4 fällt (a.p.-Projektion). Die Röntgenröhre 2 und der Röntgenbildverstärker 6 sind beispielsweise derart schräg angeordnet, daß der Zentralstrahl der Röntgenröhre 2 den Zentralstrahl der Röntgenröhre 1 in einem Zielgebiet innerhalb des Patienten 4 unter einem Winkel $\alpha$ von beispielsweise 45° schneidet (c.c.-Projektion). Dadurch erhält man Durchleuchtungsbilder aus zwei verschiedenen Projektionsrichtungen, so daß man den Patienten 4 durch die Patientenliege 3 derart verschieben kann, daß sich beispielsweise ein Nierenstein in dem Zielgebiet einer Vorrichtung zum Zertrümmern von Konkrementen befindet.

Die Ausgangssignale der an den Röntgenbildverstärker 5 und 6 angekoppelten Fernsehkameras 7 und 8 werden in zwei Bildspeichern 9 und 10 eingelesen. Die Ausgangssignale der Bildspeicher 9 und 10 werden einer Verarbeitungsschaltung 11 und 12 zugeführt, die beispielsweise eine Filterung der Signale bewirkt. Die Ausgänge der Verarbeitungsschaltung 11 und 12 sind an je einem Bildrechner 13 und 14 und je einem ersten Eingang einer Additionsstufe 15 und 16 angeschlossen. Die Ausgänge der Bildrechner 13 und 14 sind mit den zweiten Eingängen der jeweiligen anderen Additionsstufen 16 und 15 verbunden. An den Additionsstufen 15 und 16 ist je ein Monitor 17 und 18 angeschlossen.

Nach erfolgter Durchleuchtung durch die Röntgenröhren 1 und 2 werden die Röntgenstrahlenbilder von den Bildwandlern, den Bildverstärkern 5 und 6 sowie den angekoppelten Fernsehkameras 7 und 8 in Bildsignale umgewandelt, die in den Bildspeichern 9 und 10 eingespeichert werden. Diese gespeicherten Bildsignale werden den jeweiligen Bildrechnern 13 und 14 zugeführt. Der erste Bildrechner 13 berechnet aus dem Bildsignal, das einer a.p.-Projektion entspricht, ein Bildsignal, welches einer c.c.-Projektion entspricht, durch Drehung des a.p.-Projektionsbildes um den Winkel - $\alpha$. Dieses Ausgangssignal des ersten Bildrechners 13 wird als errechnetes Bildsignal einer c.c.-Projektion der Additionsstufe 16 zugeführt und dort mit dem in dem ersten Bildspeicher 10 enthaltenen Bildsignal der c.c.-Projektion gemischt und auf dem Monitor 18 wiedergegeben.

In gleicher Weise wird das Ausgangssignal des zweiten Bildspeichers 12, das einer c.c.-Projektion entspricht, in dem zweiten Bildrechner 14 durch Drehung des c.c.-Projektionsbildes um den Winkel $\alpha$ in ein a.p.-Projektionsbild umgewandelt, das zusammen mit dem im Bildspeicher 9 gespeicherten Bildsignal der a.p.-Projektion in der Additionsstufe 15 gemischt und auf dem Monitor 17 wiedergegeben wird. Dadurch werden zwei getrennt ermittelte Videosignale gemischt, so daß sich der Kontrast des zu untersuchenden Objektes, beispielsweise der Konkremente, erhöht. Dadurch lassen sich diese Konkremente auch dann noch erkennen, wenn sie bereits durch mehrere Stoßwellen zerkleinert wurden.

Die Mischung der gespeicherten und der errechneten Signale in den Additionsstufen 15 und 16 kann beispielsweise durch Mittelwertbildung, d.h. Addition und anschließende Division durch Zwei, erfolgen. Dadurch wird insbesondere das Rauschen verringert, so daß sich die Erkennbarkeit erhöht. Es kann aber auch nur eine Addition durchgeführt werden, wobei anschließend aus dem Summensignal ein einstellbarer Fensterbereich ausgeblendet wird, der dann auf dem Monitor 17 und 18 wiedergegeben wird. Dadurch läßt sich insbesondere der Kontrast der Konkremente erhöhen.

**Patentansprüche**

1. Röntgenortungseinrichtung mit zwei Röntgenstrahlenquellen (1, 2) zur Erzeugung zweier sich in einem Zielgebiet schneidender Röntgenstrahlenbündel und zwei Bildwandlern (5 bis 8) zur Erzeugung zweier Bildsignale und zwei daran angeschlossenen Monitoren (17, 18), dadurch gekennzeichnet, daß das Bildsignal des ersten Bildwandlers (5, 7) einem Bildrechner (13) zugeführt wird, der ein Bildsignal berechnet, das der Projektion des einen Röntgenstrahlenbündels in der Ebene des anderen Röntgenstrahlenbündels entspricht, und daß eine Additionsstufe (16) zwischen dem zweiten Bildwandler (6, 8) und dem zweiten Monitor (18) geschaltet ist, die das Ausgangssignal des Bildrechners (13) und das des zweiten Bildwandlers (6, 8) mischt.

2. Röntgendiagnostikeinrichtung nach Anspruch 1, dadurch gekennzeichnet, daß an den beiden Bildwandlern (5 bis 8) je eine Additionsstufe (15, 16) und je ein Bildrechner (13, 14) angeschlossen sind, deren Ausgänge mit der jeweils anderen Additionsstufe (15, 16) verbunden sind.

Claims

1. X-ray location device with two X-ray sources (1, 2) for the production of two X-ray beams, intersecting in a target area, and two image converters (5 to 8) for the production of two image signals and two monitors (17, 18) attached thereto, characterized in that the image signal of the first image converter (5, 7) is supplied to an image computer (13), which calculates an image signal, which corresponds to the projection of the one X-ray beam in the plane of the other X-ray beam, and in that an addition stage (16) is connected between the second image converter (6, 8) and the second monitor (18), which mixes the output signal of the image computer (13) and that of the second image converter (6, 8).

2. X-ray diagnostic device according to claim 1, characterized in that there are attached to both image converters (5 to 8) a respective addition stage (15, 16) and a respective image computer (13, 14), the outputs of which are connected to the respective other addition stage (15, 16).

**Revendications**

1. Dispositif de localisation radioscopique comportant deux sources (1, 2) de rayons X servant à produire deux faisceaux de rayons X, qui se coupent dans une zone cible, et deux convertisseurs d'images (5 à 8) servant à produire deux signaux d'images et deux moniteurs (17, 18) raccordés à ces convertisseurs, caractérisé par le fait que le signal d'image du premier convertisseur d'images (5, 7) est envoyé à un calculateur d'images (13), qui calcule un signal d'image correspondant à la projection d'un faisceau de rayons X dans le plan de l'autre faisceau de rayons X, et qu'entre le second convertisseur d'images (6, 8) et le second moniteur (18) est branché un étage additionneur (16), qui mélange le signal de sortie du calculateur d'images (13) et le signal de sortie du second convertisseur d'images (6, 8).

2. Dispositif de radiodiagnostic suivant la revendication 1, caractérisé par le fait qu'aux deux convertisseurs d'images (5 à 8) sont raccordés respectivement, étage additionneur (15, 16) et respectivement un calculateur d'images (13, 14), dont les sorties sont reliées à l'autre étage additionneur respectif (15, 16).